(19) Europäisches Patentamt
European Patent Office
Office européen des brevets

(11) **EP 3 287 003 B1**

(12) **EUROPEAN PATENT SPECIFICATION**

(45) Date of publication and mention
of the grant of the patent:
**24.06.2020 Bulletin 2020/26**

(51) Int Cl.:
*H04N 7/18* (2006.01)          *G06K 9/46* (2006.01)
*G06K 9/20* (2006.01)          *G06T 7/00* (2017.01)
*G06K 9/00* (2006.01)          *G01N 33/00* (2006.01)
*G01N 21/84* (2006.01)          *G01N 21/27* (2006.01)
*A01G 7/00* (2006.01)

(21) Application number: **16783028.0**

(22) Date of filing: **08.04.2016**

(86) International application number:
**PCT/JP2016/061521**

(87) International publication number:
**WO 2016/171007 (27.10.2016 Gazette 2016/43)**

(54) **INSPECTION DEVICE, INSPECTION METHOD, AND PROGRAM**

PRÜFVORRICHTUNG, PRÜFVERFAHREN UND PROGRAMM

DISPOSITIF D'INSPECTION, PROCÉDÉ D'INSPECTION, ET PROGRAMME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.04.2015 JP 2015089013**

(43) Date of publication of application:
**28.02.2018 Bulletin 2018/09**

(73) Proprietor: **Sony Corporation**
**Tokyo 108-0075 (JP)**

(72) Inventors:
• **TAKASHIMA, Masatoshi**
**Tokyo 108-0075 (JP)**
• **OGAWA, Tetsu**
**Tokyo 108-0075 (JP)**
• **MURAKAMI, Yoshihiro**
**Tokyo 108-0075 (JP)**
• **MATSUI, Akira**
**Tokyo 108-0075 (JP)**

(74) Representative: **D Young & Co LLP**
**120 Holborn**
**London EC1N 2DY (GB)**

(56) References cited:
WO-A1-99/19824          WO-A1-2007/000999
WO-A1-2012/053877          WO-A2-01/33505
JP-A- 2003 339 238          JP-A- 2003 339 238
JP-A- 2007 293 558          JP-A- 2007 293 558
JP-A- 2013 101 428          JP-A- 2013 242 624
JP-A- 2013 242 624          JP-A- 2014 183 788
US-A1- 2009 136 125

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

[Technical Field]

The present disclosure relates to an inspection apparatus, an inspection method, and a program, and relates, in particular, to an inspection apparatus, an inspection method, and a program that allow for more accurate inspection results to be acquired.

[Background Art]

An inspection apparatus is hitherto known that inspects vegetation such as state and activity of a plant raised at a certain location (refer, for example, to PTL 1) . JP 2003 339238 A discloses an apparatus with Image acquisition means which overlaps an image frame and acquires some images. Image-analysis means carries out the comparison analysis of the picture information from which the angle of depression of the same point of said overlapped part differs. A variation of the picture information resulting from the appearance vegetation degree which changes by an angle of depression is corrected.

[Citation List]

[Patent Literature]

[PTL 1]
JP 2003-9664 A

[Summary]

[Technical Problem]

However, it was difficult to acquire accurate inspection results with such an inspection apparatus.
The present disclosure has been devised in light of the foregoing, and it is an object of the disclosure to allow for acquisition of more accurate inspection results.

[Technical Solution]

In a first aspect of the present disclosure, there is provided a vegetation inspection apparatus comprising: a calculation process section configured to calculate an inspection value for inspecting plant vegetation by using imaging data having conflicting inspection-related-angle-dependent components such that the inspection-related-angle-dependent components included in imaging data acquired by imaging the plant vegetation are reduced; and an imaging control section configured to control an imaging apparatus for imaging the plant vegetation, wherein the imaging control section causes the imaging apparatus to image the plant vegetation at timings that are in accordance with imaging directions of imaging the plant vegetation and shining directions of light shone on the plant vegetation.

In a second aspect of the present disclosure, there is provided a vegetation inspection method comprising: calculating an inspection value for inspecting plant vegetation by using imaging data having conflicting inspection-related-angle-dependent components such that the inspection-related-angle-dependent components included in imaging data acquired by imaging the plant vegetation are reduced; and controlling an imaging apparatus for imaging the plant vegetation, wherein the imaging apparatus images the plant vegetation at timings that are in accordance with imaging directions of imaging the plant vegetation and shining directions of light shone on the plant vegetation.

In a third aspect of the present disclosure, there is provided a program causing a computer to function as: a calculation process section configured to calculate an inspection value for inspecting plant vegetation by using imaging data having conflicting inspection-related-angle-dependent components such that the inspection-related-angle-dependent components included in imaging data acquired by imaging the plant vegetation are reduced; and an imaging control section configured to control an imaging apparatus for imaging the plant vegetation, wherein the imaging control section causes the imaging apparatus to image the plant vegetation at timings that are in accordance with imaging directions of imaging the plant vegetation and shining directions of light shone on the plant vegetation.

In an aspect of the present disclosure, an inspection value is calculated for inspecting plant vegetation (also referred to herein as an inspection target) by using imaging data (also referred to herein as sensing data) obtained at different timings so as to have conflicting inspection-related-angle-dependent components such that the inspection-related-angle-dependent components included in sensing data acquired by sensing the inspection target are reduced.

Various inspection conditions are described herein, but any for which timing does not play a role do not fall within the scope of the invention, the scope of the invention being defined by the claims.

[Advantageous Effect of Invention]

According to an aspect of the present disclosure, it is possible to acquire more accurate inspection results.

[Brief Description of Drawings]

[Fig. 1]

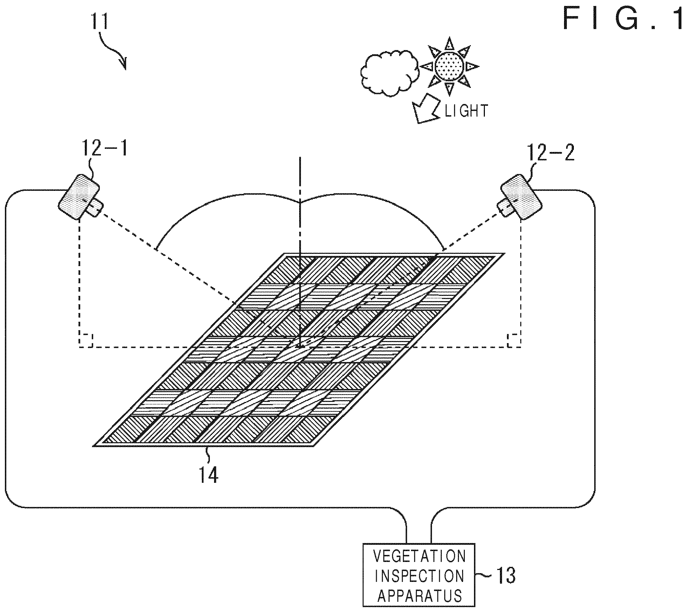

Fig. 1 is a diagram illustrating a configuration example of an embodiment of a vegetation inspection system to which the present technology is applied.
[Fig. 2]

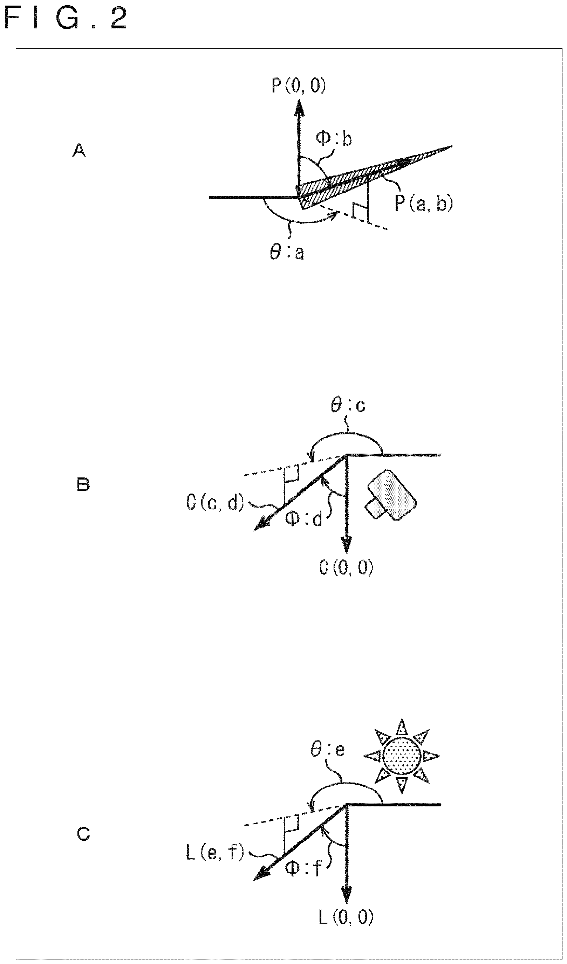

Fig. 2 is a diagram describing definitions of a grain angle, an imaging direction angle, and a shining direction angle.

[Fig. 3]

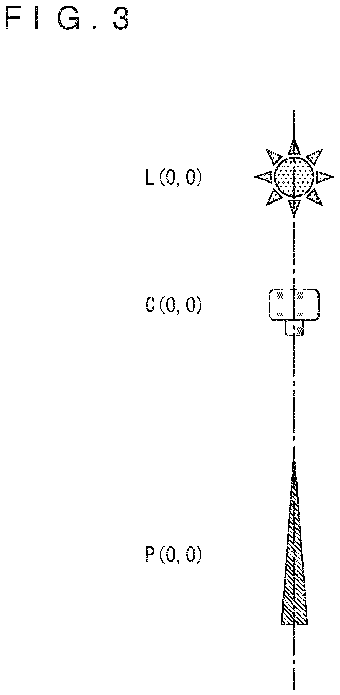

Fig. 3 is a diagram describing a state free from angle-dependent effects.

[Fig. 4]

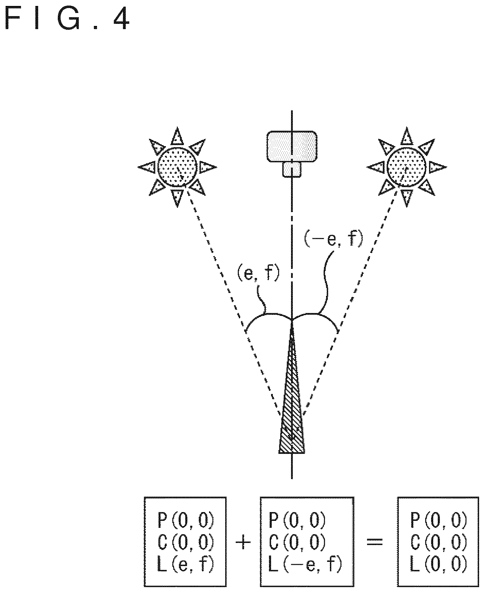

Fig. 4 is a diagram describing a first inspection condition.

[Fig. 5]

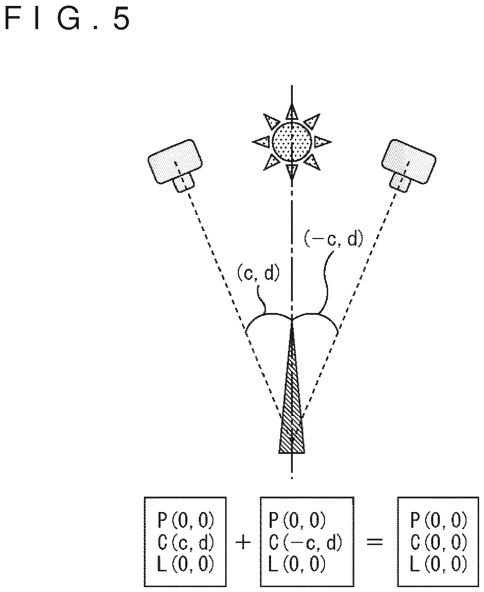

Fig. 5 is a diagram describing a second inspection condition.

[Fig. 6]

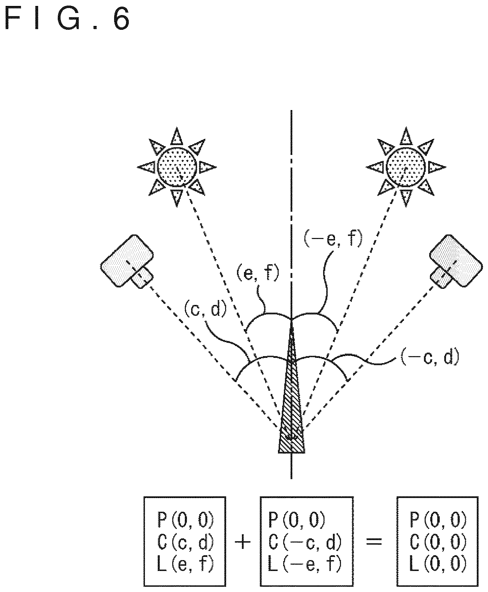

Fig. 6 is a diagram describing a third inspection condition.

[Fig. 7]

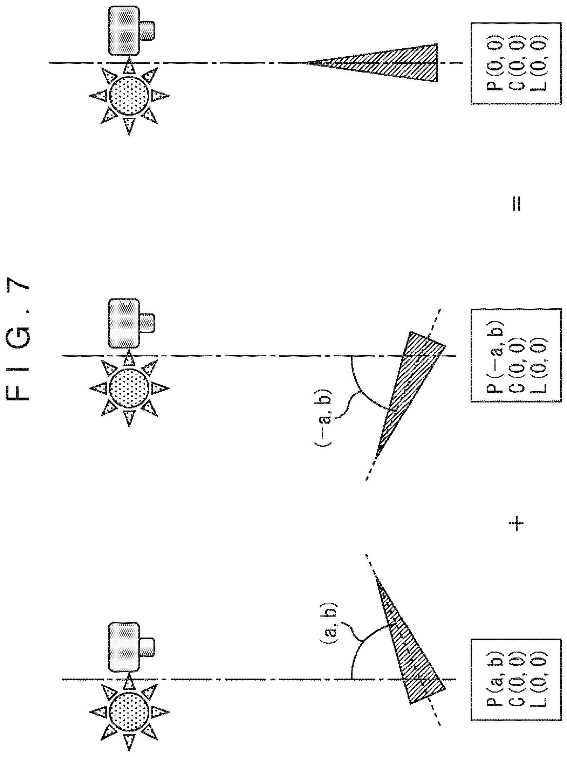

Fig. 7 is a diagram describing a fourth inspection condition.

[Fig. 8]

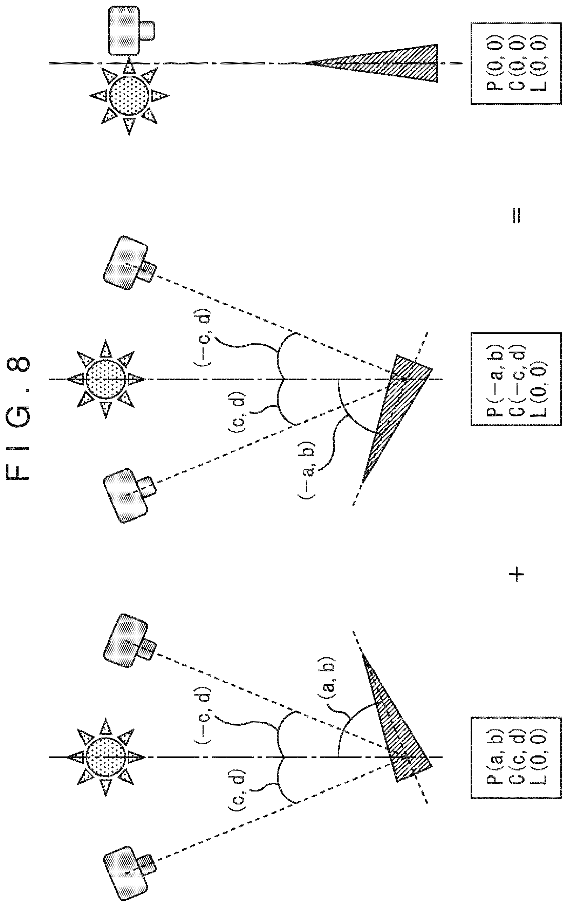

Fig. 8 is a diagram describing a fifth inspection condition.

[Fig. 9]

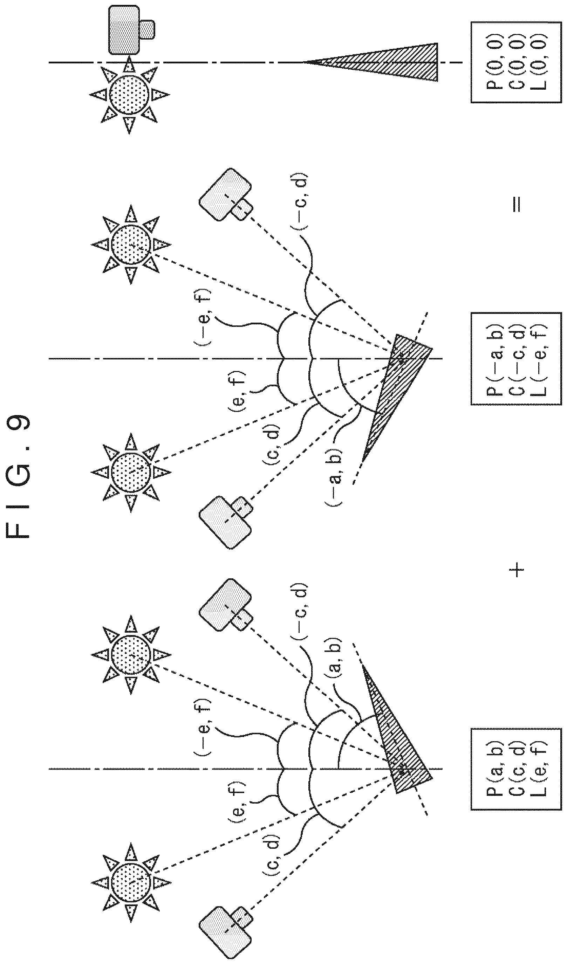

Fig. 9 is a diagram describing a sixth inspection condition.

[Fig. 10]

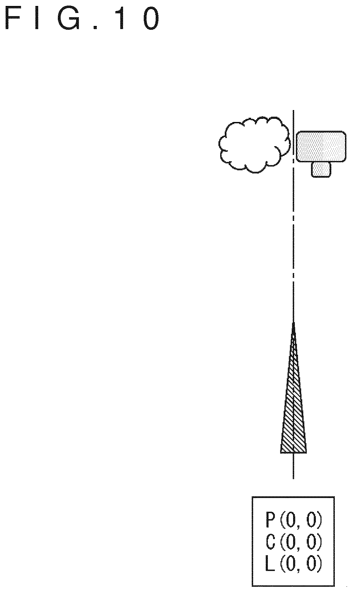

Fig. 10 is a diagram describing a seventh inspection condition.

[Fig. 11]

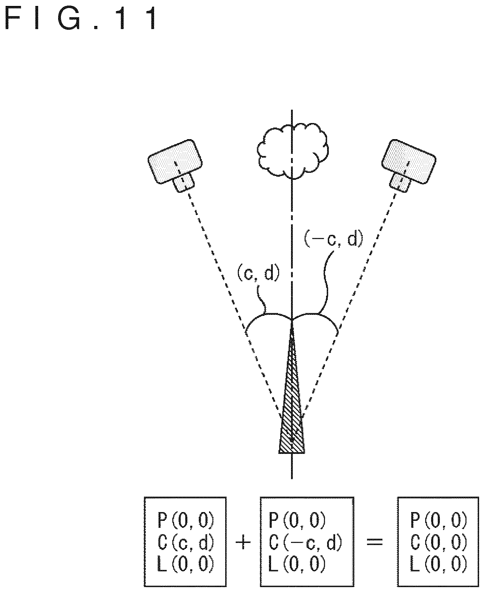

Fig. 11 is a diagram describing an eighth inspection condition.

[Fig. 12]

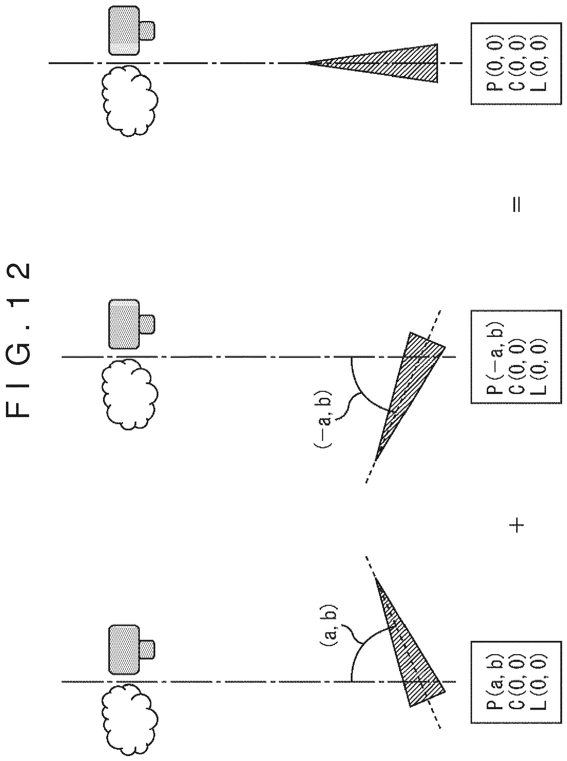

Fig. 12 is a diagram describing a ninth inspection condition.

[Fig. 13]

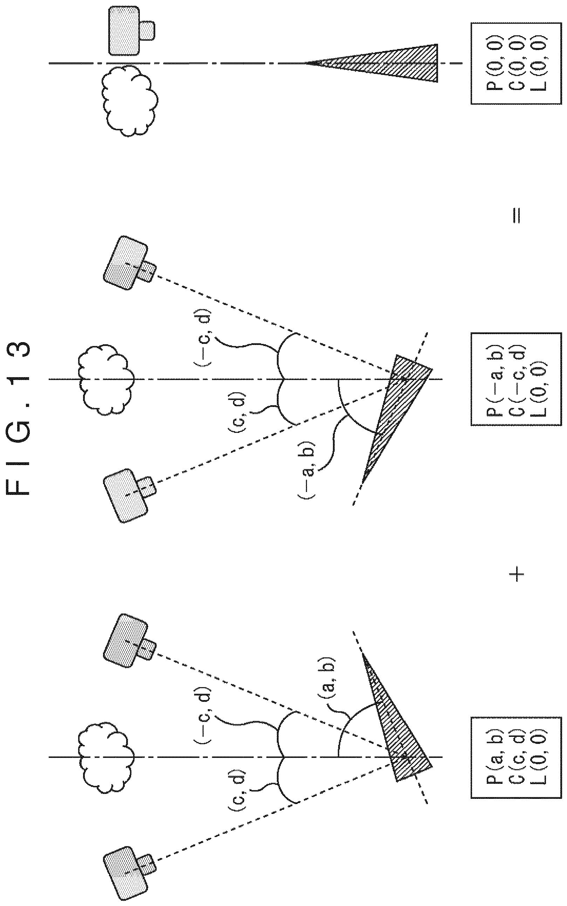

Fig. 13 is a diagram describing a tenth inspection condition.

[Fig. 14]

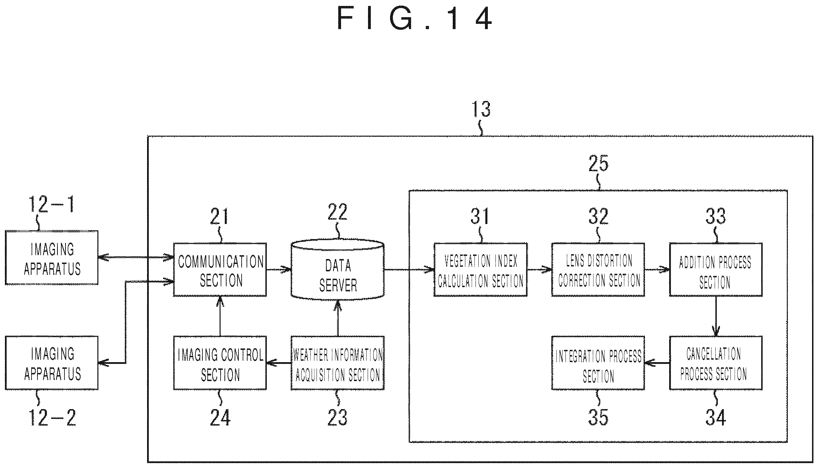

Fig. 14 is a block diagram illustrating a configuration example of a vegetation inspection apparatus.

[Fig. 15]

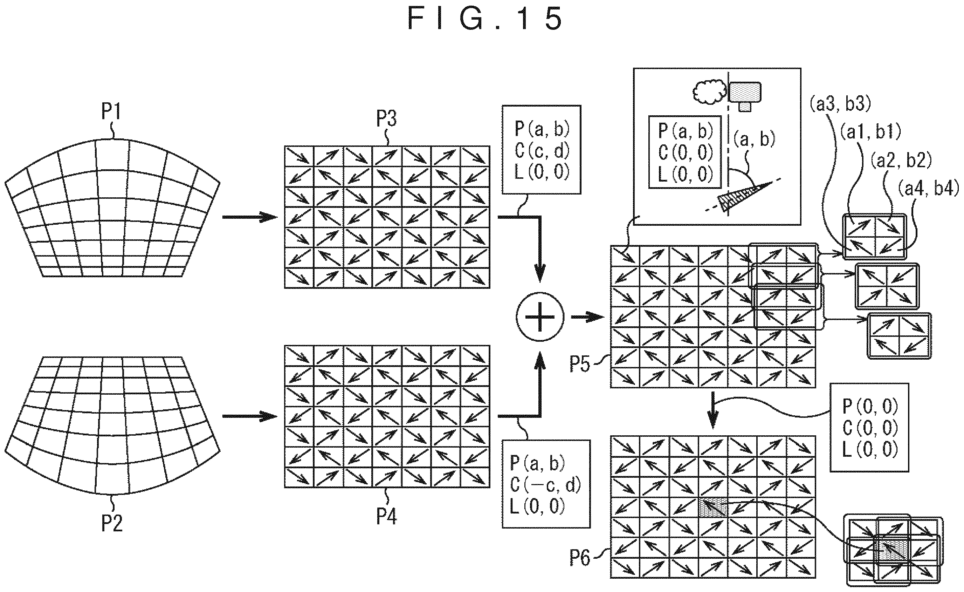

Fig. 15 is a diagram describing a process performed by a calculation process section.

[Fig. 16]

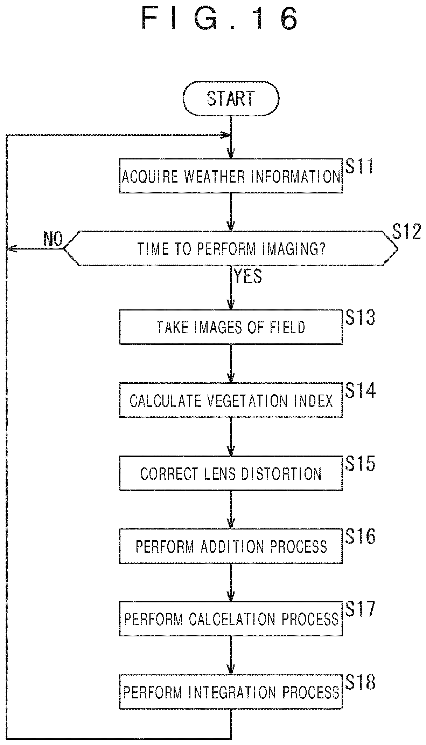

Fig. 16 is a flowchart describing an example of a process for finding a vegetation index.

[Fig. 17]

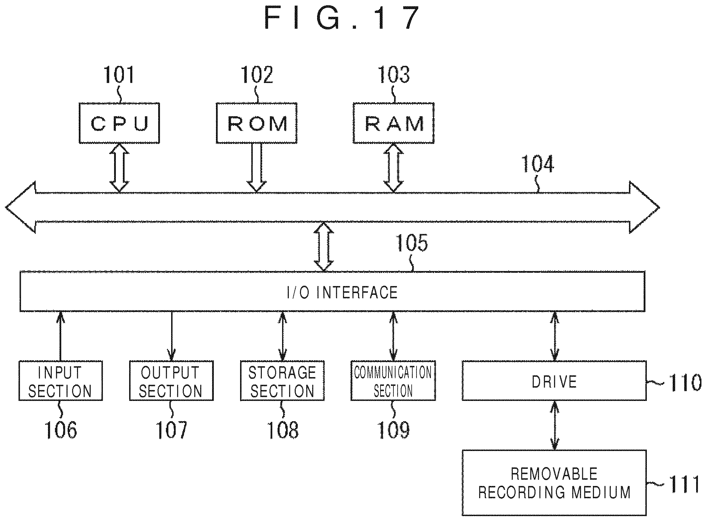

Fig. 17 is a block diagram illustrating a configuration example of an embodiment of a computer to which the present technology is applied.

[Description of Embodiment]

A detailed description will be given below of an embodiment of a vegetation inspection system to which the present technology is applied with reference to drawings.

<Embodiment of Vegetation Inspection System>

A description will be given of a configuration example of an embodiment of a vegetation inspection system to which the present technology is applied with reference to Fig. 1.

As illustrated in Fig. 1, a vegetation inspection system 11 is configured to include two imaging apparatuses 12-1 and 12-2 and a vegetation inspection apparatus 13. Then, the vegetation inspection system 11 inspects, for example, plant vegetation, targeting various plants such as lawn, rice, and sugar canes raised in a field 14 as inspection targets. Also, during inspection of plant vegetation by the vegetation inspection system 11, sunlight is shone on the field 14 or obstructed by clouds.

The imaging apparatuses 12-1 and 12-2 are fastened in accordance with given arrangement conditions with respect to the field 14 and communicate with the vegetation inspection apparatus 13 via a wiredly or wirelessly constructed communication network. Then, each of the imaging apparatuses 12-1 and 12-2 takes an image of the field 14 under control of the vegetation inspection apparatus 13 and sends the image of the field 14 acquired as a result thereof to the vegetation inspection apparatus 13.

For example, the imaging apparatuses 12-1 and 12-2 are fastened under the arrangement conditions in which the imaging apparatuses 12-1 and 12-2 are located at the same distance from the center of the field 14 on a straight line that passes the center of the field 14 and in which the imaging apparatuses 12-1 and 12-2 are at the same height from the field 14 and at the same elevation angle toward the center of the field 14. That is, as illustrated in Fig. 1, the imaging apparatuses 12-1 and 12-2 are fastened in such a manner as to face the azimuths opposite to each other so that angles formed by optical axes of the imaging apparatuses 12-1 and 12-2 relative to the perpendicular line on the center of the flat field 14 are the same.

The vegetation inspection apparatus 13 controls timings when the imaging apparatuses 12-1 and 12-2 take images of the field 14. Then, the vegetation inspection apparatus 13 finds a vegetation index for inspecting vegetation of a plant raised in the field 14 on the basis of images of the field 14 taken by the imaging apparatuses 12-1 and 12-2. It should be noted that the detailed configuration of the vegetation inspection apparatus 13 will be described later with reference to Fig. 14.

The vegetation inspection system 11 configured as described above can find a vegetation index free from (with reduced) angle-dependent effect for each of directions in which the plant raised in the field 14 grows, in which the imaging apparatuses 12-1 and 12-2 take images of the field 14, in which light is shone on the field 14 from the sun.

For example, in a case where a lawn is raised in the field 14, the direction in which the lawn grows (hereinafter referred to as the grain) varies significantly depending on mowing thereof. Therefore, it is difficult to constantly inspect vegetation under the same conditions. For this reason, a description will be given below assuming that a lawn is inspected by the vegetation inspection system 11 in the field 14 where the lawn as can be found in a soccer stadium is raised. Of course, not only the above-described rice and sugar canes but also various other plants can be subject to inspection by the vegetation inspection system 11.

For example, hatching directions of the field 14 in Fig. 1 indicate the grains of the lawn raised in the field 14, and the vegetation inspection system 11 is capable of inspecting vegetation in a manner free from any effect of the grain angle of the lawn that is raised such that the grain differs from one given area to another. It should be noted that each of the imaging apparatuses 12-1 and 12-2 will be simply referred to as the imaging apparatuses 12 as appropriate in a case where it is not necessary to distinguish between the imaging apparatuses 12-1 and 12-2.

<Definitions of Azimuth Angle and Elevation Angle>

A description will be given of the grain angle in the field 14, the angle of the imaging direction by the imaging apparatuses 12, and the angle of the shining direction of light shone on the field 14 with reference to Fig. 2.

As illustrated in A of Fig. 2, for example, a grain angle P in the field 14 is defined on the basis of an azimuth angle $\theta$ of the grain with respect to a given reference direction and an elevation angle $\varphi$ of the grain with respect to a vertically upward direction. That is, in a case where the azimuth angle $\theta$ of the grain is "a" and the elevation angle $\varphi$ of the grain is "b," the grain angle is defined as P(a,b). It should be noted that in a case where the grain points vertically upward, the grain angle is P(0,0).

Also, as illustrated in B of Fig. 2, an angle C of an imaging direction by the imaging apparatuses 12 is defined on the basis of the azimuth angle $\theta$ of the imaging direction with respect to a given reference direction and the elevation angle $\varphi$ of the imaging direction with respect to a vertically downward direction. That is, in a case where the azimuth angle $\theta$ of the imaging direction is "c" and the elevation angle $\varphi$ of the imaging direction is "d," the imaging direction angle is defined as C(c,d). It should be noted that in a case where the imaging direction points vertically downward, the imaging direction angle is C(0,0).

Similarly, as illustrated in C of Fig. 2, an angle L of the direction of light shone on the field 14 is defined on the basis of the azimuth angle $\theta$ of the shining direction with respect to a given reference direction and the elevation angle $\varphi$ of the shining direction with respect to the vertically downward direction. That is, in a case where the azimuth angle $\theta$ of the shining direction is "e" and the elevation angle $\varphi$ of the shining direction is "f," the shining direction angle is defined as L(e,f). It should be noted that in a case where the shining direction points vertically downward, the shining direction angle is L(0,0).

Then, when the grain angle P(0,0), the imaging direction angle C(0,0), and the shining direction angle L(0,0) are all satisfied, inspection can be conducted in a manner free from effects dependent upon these angles as illustrated in Fig. 3.

However, it is difficult to conduct inspection such that the grain angle P(0,0), the imaging direction angle C(0,0), and the shining direction angle L(0,0) are all satisfied. For this reason, the vegetation inspection system 11 is capable of eliminating effects dependent upon these angles using inspection conditions as illustrated with reference to Figs. 4 to 13. That is, the grain angle, the imaging direction angle, and the shining direction angle are angles related to inspection of the field 14 by the vegetation inspection apparatus 13, and effects dependent upon these angles manifest themselves in images taken by the imaging apparatuses 12. Therefore, the vegetation inspection system 11 conducts vegetation inspection by using images taken under inspection conditions where the components dependent upon these inspection-related angles (e.g., azimuth angle $\theta$, elevation angle $\varphi$) conflict with each other.

<Inspection Conditions That Eliminate Angle-Dependent Effects>

A description will be given of a first inspection condition for eliminating angle-dependent effects with reference to Fig. 4.

As illustrated in Fig. 4, when the grain angle is P(0,0) and the imaging direction angle is C(0,0), it is possible to eliminate angle-dependent effects by performing imaging twice, with the shining direction angles L(e,f) and L(-e,f). That is, in the first inspection condition, the grain points vertically upward, and imaging is performed vertically downward from directly above twice when the light shining directions are at symmetrical angles relative to the vertical direction (at opposite azimuth angles).

By taking the arithmetic mean of the two images taken under the inspection condition described above, the difference in shining direction angle is cancelled (the shining direction angle becomes equivalent to L(0,0)), making it possible to eliminate the effect dependent upon the shining direction angle.

A description will be given of a second inspection condition for eliminating angle-dependent effects with reference to Fig. 5.

As illustrated in Fig. 5, when the grain angle is P(0,0) and the shining direction angle is L(0,0), it is possible to eliminate angle-dependent effects by performing imaging from two directions where the imaging direction angles are C(c,d) and C(-c,d). That is, in the second inspection condition, the grain points vertically upward, and imaging is performed from two directions where the imaging directions are at symmetrical angles relative to the vertical direction when light is shone vertically downward from directly above.

By taking the arithmetic mean of the two images taken under the second inspection condition described above, the difference in imaging direction angle is cancelled (the imaging direction angle becomes equivalent to C(0,0)), making it possible to eliminate effects dependent upon the imaging direction angle.

A description will be given of a third inspection condition for eliminating angle-dependent effects with reference to Fig. 6.

As illustrated in Fig. 6, it is possible to eliminate angle-dependent effects by performing first imaging with the shining direction angle L(e,f) and the imaging direction angle C(c,d) and performing second imaging with the shining direction angle L(-e,f) and the imaging direction angle C(-c,d) when the grain angle is P(0,0). That is, in the third inspection condition, the grain points vertically upward, and imaging is performed from two directions where the imaging directions are at symmetrical angles relative to the vertical direction and twice, and each time when the light shining directions are at symmetrical angles relative to the vertical direction.

By taking the arithmetic mean of the two images taken under the third inspection condition described above, the differences in imaging direction angle and shining direction angle are cancelled (the imaging direction angle becomes equivalent to C(0,0) and the shining direction angle becomes equivalent to L(0,0)), making it possible to eliminate effects dependent upon the imaging direction angle and the shining direction angle.

A description will be given of a fourth inspection condition for eliminating angle-dependent effects with reference to Fig. 7.

As illustrated in Fig. 7, it is possible to eliminate angle-dependent effects by taking images of two areas where the grain angles are P(a,b) and P(-a,b) when the shining direction angle is L(0,0) and the imaging direction angle is C(0,0). That is, in the fourth inspection condition, imaging is performed vertically downward when light is shone vertically downward to take images of two areas where the grain is at symmetrical angles relative to the vertically upward direction.

By taking the arithmetic mean of the two images taken under the fourth inspection condition described above, the difference in grain angle is cancelled (the grain angle becomes equivalent to P(0,0)), making it possible to eliminate the effect dependent upon the grain angle. As will be described later, for example, in a case where two areas whose grain angle components conflict with each other are included in a single image, it is possible to reduce the effect dependent upon the grain angle by taking the arithmetic mean of sensing values of two areas as sensing data.

A description will be given of a fifth inspection condition for eliminating angle-dependent effects with reference to Fig. 8.

As illustrated in Fig. 8, when the shining direction angle is L(0,0), it is possible to eliminate angle-dependent effects by taking an image of an area having the grain angle P(a,b) at the imaging direction angle C(c,d) and taking an image of an area having the grain angle P(-a,b) at the imaging direction angle C(-c,d). That is, in the fifth inspection condition, imaging is performed from two directions at symmetrical angles relative to the vertically downward direction to take images of two respective areas where the grain is at symmetrical angles relative to the vertically upward direction when light is shone vertically downward.

By taking the arithmetic mean of the two images taken under the fifth inspection condition described above, the differences in grain angle and imaging direction angle are cancelled (the grain angle becomes equivalent to P(0,0) and the imaging direction angle becomes equivalent to C(0,0)), making it possible to eliminate the effects dependent upon the grain angle and imaging direction angle.

A description will be given of a sixth inspection condition for eliminating angle-dependent effects with reference to Fig. 9.

As illustrated in Fig. 9, it is possible to eliminate angle-dependent effects by performing first imaging to take an image of an area having the grain angle P(a,b) from the imaging direction angle C(c,d) when the shining direction angle reaches L(e,f) and performing second imaging to take an image of an area having the grain angle P(-a,b) from the imaging direction angle C(-c,d) when the shining direction angle reaches L(-e,f). That is, in the sixth inspection condition, imaging is performed from two directions at symmetrical angles relative to the vertically downward direction to take images of two respective areas where the grain is at symmetrical angles relative to the vertically upward direction, and imaging is performed at two timings when the light shining directions are at symmetrical angles relative to the vertical direction.

By taking the arithmetic mean of the two images taken under the sixth inspection condition described above, the differences in grain angle, imaging direction angle, and shining direction are cancelled (the grain angle becomes equivalent to P(0,0), and the imaging direction angle becomes equivalent to C(0,0), and the shining direction angle becomes equivalent to L(0,0)), making it possible to eliminate the effects dependent upon the grain angle, imaging direction angle, and shining direction angle.

Incidentally, in Figs. 4 to 9, a description has been given of situations in which effects dependent upon the angle of shining direction of light shone on the lawn are sustained, that is, situations in which the sun is out during daytime. It should be noted, however, that there are only limited locations where the sun comes overhead, and it is presumably difficult to completely eliminate effects dependent upon the shining direction angle by using an east/west combination alone. For this reason, in order to eliminate effects dependent upon the shining direction angle, it is possible to make use of the condition in which there is no direct shining of sunlight, that is, the condition in which light is diffused in cloudy or rainy weather, before the sunrise, after the sunset, and so on.

That is, in a case where the weather is cloudy with no sunlight directly shone on the field 14, there is no need to consider the effect dependent upon the shining direction angle (it is acceptable to consider that the shining direction is equivalent to angle L(0,0)). In seventh to tenth inspection conditions for eliminating angle-dependent effects which will be described below, imaging is performed in cloudy weather.

A description will be given of a seventh inspection condition for eliminating angle-dependent effects with reference to Fig. 10.

As illustrated in Fig. 10, it is possible to eliminate angle-dependent effects by performing imaging once at the imaging direction angle C(0,0) when the grain angle is P(0,0). That is, in the seventh inspection condition, the grain points vertically upward, and imaging is performed vertically downward from directly above. That is, in this case, it is possible to eliminate angle-dependent effects using images acquired by a single occasion of imaging.

A description will be given of an eighth inspection condition for eliminating angle-dependent effects with reference to Fig. 11.

As illustrated in Fig. 11, when the grain angle is P(0,0), it is possible to eliminate angle-dependent effects by performing imaging from two directions where the imaging direction angles are C(c,d) and C(-c,d). That is, in the eighth inspection condition, the grain points vertically upward, and imaging is performed from two directions where the imaging directions are at symmetrical angles relative to the vertical direction.

By taking the arithmetic mean of the two images taken under the eighth inspection condition described above, the difference in imaging direction angle is cancelled (the imaging direction angle becomes equivalent to C(0,0)), making it possible to eliminate the effect dependent upon the imaging direction angle.

A description will be given of a ninth inspection condition for eliminating angle-dependent effects with reference to Fig. 12.

As illustrated in Fig. 12, when the imaging direction angle is C(0,0), it is possible to eliminate the angle-dependent effect by taking images of two areas where the grain angles are P(a,b) and P(-a,b). That is, in the ninth inspection condition, imaging is performed vertically downward to take images of two areas where the grain is at symmetrical angles relative to the vertically upward direction.

By taking the arithmetic mean of the two images taken under the ninth inspection condition described above, the difference in grain angle is cancelled (the grain angle becomes equivalent to P(0,0)), making it possible to eliminate the effect dependent upon the grain angle.

A description will be given of a tenth inspection condition for eliminating angle-dependent effects with reference to Fig. 13.

As illustrated in Fig. 13, it is possible to eliminate angle-dependent effects by taking an image of an area having the grain angle P(a,b) at the imaging direction angle C(c,d) and taking an image of an area having the grain angle P(-a,b) at the imaging direction angle C(-c,d). That is, in the tenth inspection condition, imaging is performed from two directions at symmetrical angles relative to the vertically downward direction to take images of two respective areas where the grain is at symmetrical angles relative to the vertically upward direction.

By taking the arithmetic mean of the two images taken under the tenth inspection condition described above, the differences in grain angle and imaging direction angle are cancelled (the grain angle becomes equivalent to P(0,0) and the imaging direction angle becomes equivalent to C(0,0)), making it possible to eliminate the effects dependent upon the grain angle and imaging direction angle.

<Configuration Example of Vegetation Inspection Apparatus>

Fig. 14 is a block diagram illustrating a configuration example of the vegetation inspection apparatus 13.

As illustrated in Fig. 14, the vegetation inspection apparatus 13 is configured to include a communication section 21, a data server 22, a weather information acquisition section 23, an imaging control section 24, and a calculation process section 25, and the imaging apparatuses 12-1 and 12-2 are connected to the vegetation inspection apparatus 13. Here, the imaging apparatuses 12-1 and 12-2 are configured to include, for example, a sensor that has not only detection devices for each detecting red, green, and blue light in the visible range but also detection devices for detecting near-infrared (NIR) outside the visible range. The detection devices are arranged two-dimensionally. The imaging apparatuses 12-1 and 12-2 are used as sensing apparatuses for sensing light from the field 14. That is, the imaging apparatuses 12-1 and 12-2 include an imaging sensor that has pixels (detection devices) arranged two-dimensionally, for detecting light in different wavelength ranges for the respective wavelength ranges, and images for the respective wavelength ranges taken with the imaging sensor (image data including sensing values) are an example of sensing data acquired by sensing.

The communication section 21 communicates with the imaging apparatuses 12-1 and 12-2. For example, the communication section 21 receives image data making up images taken with the imaging apparatuses 12-1 and 12-2 (e.g., Raw data made up of red (R), green (G), blue (B), and infrared (IR) pixel values) and supplies the image data to the data server 22. Also, when supplied with an imaging command instructing the imaging apparatuses 12-1 and 12-2 to perform imaging from the imaging control section 24, the communication section 21 sends the imaging command to the imaging apparatuses 12-1 and 12-2.

The data server 22 accumulates image data supplied from the communication section 21 and supplies image data to the calculation process section 25 in response to a request from the calculation process section 25. The data server 22 also acquires weather information at the time of image taking by the imaging apparatuses 12-1 and 12-2 via the weather information acquisition section 23 and stores the weather information in association with image data corresponding to the image.

The weather information acquisition section 23 acquires, for example, weather information observed by an observational instrument (not illustrated) installed in the field 14 or weather information in the neighborhood of the field 14 delivered via an external network such as the Internet and supplies such weather information to the imaging control section 24 as required. The weather information acquisition section 23 also supplies, to the data server 22, weather information at the time of taking of image data accumulated in the data server 22.

The imaging control section 24 sends an imaging command instructing the imaging apparatuses 12-1 and 12-2 to perform imaging to the imaging apparatuses 12-1 and 12-2 via the communication section 21 in accordance with time information measured by a built-in timer (e.g., data including date, hours, minutes, and seconds) or weather information supplied from the weather information acquisition section 23. As a result, the imaging control section 24 controls timings when the imaging apparatuses 12-1 and 12-2 image the field 14.

For example, the imaging control section 24 sends an imaging command when the angle of shining direction of light shone on the field 14 reaches L(0,0) as described above with reference to Figs. 5, 7, and 8 in accordance with time information measured by the built-in timer. The imaging control section 24 also sends an imaging command when the angle of shining direction of light shone on the field 14 reaches L(e,f) and L(-e,f) as described above with reference to Figs. 4, 6, and 9 in accordance with time information measured by the built-in timer. The imaging control section 24 also sends an imaging command when the weather at the field 14 turns cloudy as described above with reference to Figs. 10 to 13 in accordance with the weather information supplied from the weather information acquisition section 23.

The calculation process section 25 reads image data accumulated in the data server 22 and calculates a vegetation index of the field 14 free from angle-dependent effects using a set of images taken with the imaging apparatuses 12-1 and 12-2. That is, the calculation process section 25 is configured to include, as illustrated, a vegetation index calculation section 31, a lens distortion correction section 32, an addition process section 33, a cancellation process section 34, and an integration process section 35.

When image data of a set of images taken with the imaging apparatuses 12-1 and 12-2 is accumulated in the data server 22, the vegetation index calculation section 31 reads these pieces of image data from the data server 22 and calculates a vegetation index for use as an inspection value for inspecting vegetation.

For example, in a case where the vegetation inspection system 11 uses a normalized difference vegetation index (NDVI), an index indicating vegetation distribution condition and activity, the normalized difference vegetation index NDVI can be calculated by computing the following Formula (1). As illustrated in Formula (1), the normalized difference vegetation index NDVI can be found by using a pixel value R representing the red component in the visible range and a pixel value IR representing the component in the near-infrared range.

[Formula 1]

$$NDVI = IR-R/IR+R \qquad \cdots (1)$$

Therefore, the vegetation index calculation section 31 finds the normalized difference vegetation index NDVI for each of the pixels making up each of the images by using the pixel value R and the pixel value IR of the image data acquired by imaging of the field 14 by the respective imaging apparatuses 12-1 and 12-2. Then, the vegetation index calculation section 31 generates two NDVI images on the basis of the pixel values of the two images taken with the imaging apparatuses 12-1 and 12-2.

The lens distortion correction section 32 corrects lens distortion of the two NDVI images generated by the vegetation index calculation section 31.

That is, as illustrated in Fig. 1, lens distortion is present in the images taken from the imaging directions at given elevation angles relative to the field 14 when a wide-angle lens (e.g., fish-eye lens) was attached to the imaging apparatuses 12-1 and 12-2 to take images of the entire field 14. Therefore, similar lens distortion is present in the NDVI images generated on the basis of such images. For example, the straight lines in the field 14 are curved in the peripheral areas of the NDVI images. Therefore, the lens distortion correction section 32 corrects the lens distortion present in the NDVI images such that the straight lines in the field 14 are also straight in the peripheral areas of the NDVI images.

The addition process section 33 performs a process of taking the arithmetic mean of the two NDVI images whose lens distortion has been corrected by the lens distortion correction section 32, generating a single NDVI image with cancelled difference in imaging direction angle.

In the cancellation process section 34, information is registered in advance about the azimuth angle of the lawn for each of the areas distinguished by grain of the lawn raised in the field 14 (e.g., which area has what kind of grain). Then, the cancellation process section 34 cancels the difference in grain angle in the NDVI image generated by the addition process section 33 by adding the pixel values of some of the areas in the two-dimensional sensing data whose azimuth angles of the grain are opposite to each other.

The integration process section 35 performs a process of taking the mean of the area data that has been added up by the cancellation process section 34, dividing the data into areas distinguished by grain and integrating the data for the normalized difference vegetation index NDVI in which the difference in grain angle is cancelled by the cancellation process section 34.

The vegetation inspection apparatus 13 configured as described above makes it possible to acquire vegetation information (NDVI image) free from the effects dependent upon the grain angle, imaging direction angle, and shining direction angle.

A description will be given of a process performed by the calculation process section 25, for example, under the tenth inspection condition illustrated in Fig. 13 with reference to Fig. 15.

In the field 14, for example, a lawn is raised such that four areas with the different grain angles P(a,b) are arranged alternately. That is, an area with the grain angle P(a1,b1), an area with the grain angle P(a2,b2), an area with the grain angle P(a3,b3), and an area with the grain angle P(a4,b4) are arranged, two down and two across, and these four areas are laid over the entire field 14.

Then, when the weather is cloudy, the field 14 is imaged from the imaging direction at the angle C(c,d) by the imaging apparatus 12-1, and the field 14 is imaged from the imaging direction at the angle C(-c,d) by the imaging apparatus 12-2.

At this time, lens distortion is present in an NDVI image P1 generated by the vegetation index calculation section 31 from the image taken with the imaging apparatus 12-1 in accordance with the imaging direction angle C(c,d) by the imaging apparatus 12-1. Similarly, lens distortion is present in an NDVI image P2 generated by the vegetation index calculation section 31 from the image taken with the imaging apparatus 12-2 in accordance with the imaging direction angle C(-c,d) by the imaging apparatus 12-2.

Therefore, the lens distortion correction section 32 generates an NDVI image P3 with corrected lens distortion of the NDVI image P1 and generates an NDVI image P4 with corrected lens distortion of the NDVI image P2. Here, each of rectangles illustrated in a grid pattern in the NDVI images P3 and P4 represents each of the areas distinguished in accordance with the grain angle P(a,b), and each of arrows illustrated in each of the rectangles indicates the grain angle P(a,b) .

At this time, the normalized difference vegetation index NDVI of each of the pixels making up the NDVI image P3 includes a component dependent upon the grain angle P(a,b) and a component dependent upon the imaging direction angle C(c,d). Similarly, the normalized difference vegetation index NDVI of each of the pixels making up the NDVI image P4 includes a component dependent upon the grain angle P(a,b) and a component dependent upon the imaging direction angle C(-c, d) .

Then, the addition process section 33 performs a process of taking the arithmetic mean of the NDVI images P3 and P4, generating an NDVI image P5 in which the component dependent upon the imaging direction angle C(c,d) and the component dependent upon the imaging direction angle C(-c,d) have cancelled each other out. That is, the NDVI image P5 becomes equivalent to the imaging direction angle C(0,0). It should be noted that the shining direction angle is L(0,0) because imaging is performed in cloudy weather.

Next, the cancellation process section 34 performs a process of cancelling the component dependent upon the grain angle P(a,b) included in the NDVI image P5.

For example, the azimuth angles θ of the grain angle P(a1,b1) and the grain angle P(a3,b3) are opposite to each other, and the azimuth angles θ of the grain angle P(a2,b2) and the grain angle P(a4,b4) are opposite to each other. Therefore, the cancellation process section 34 can cancel the component dependent upon the grain angle P(a,b) by adding the normalized difference vegetation index NDVI of the area with the grain angle P(a1,b1), the normalized difference vegetation index NDVI of the area with the grain angle P(a2,b2), the normalized difference vegetation index NDVI of the area with the grain angle P(a3,b3), and the normalized difference vegetation index NDVI of the area with the grain angle P(a4,b4).

At this time, the cancellation process section 34 performs a process of adding the normalized difference vegetation indices NDVI such that the two of the four areas subject to addition overlap each other as illustrated on the right of the NDVI image P5.

As a result, all the components dependent upon angles are cancelled from the normalized difference vegetation index NDVI, and the normalized difference vegetation index NDVI equivalent to the grain angle P(0,0), the imaging direction angle C(0,0), and the shining direction angle L(0,0) is acquired.

Thereafter, the integration process section 35 performs a process of taking the mean of the data of the four areas, dividing the data into area-by-area information for each grain angle P(a,b) and integrating the data, outputting an NDVI image P6 made up of the normalized difference vegetation index NDVI of the entire field 14 free from angle dependence. That is, as illustrated on the right side of the NDVI image P6, assuming a certain area to be a center, the integration process section 35 takes the mean by adding up the mean of the four areas having that area at bottom right, the mean of the four areas having that area at bottom left, the mean of the four areas having that area at top right, and the mean of the four areas having that area at top left, and uses the mean as the normalized difference vegetation index NDVI of that area. The integration process section 35 performs such averaging for all areas.

This allows the integration process section 35 to output the NDVI image P6 made up of the normalized difference vegetation index NDVI of the entire field 14 free from angle dependence. It should be noted that the calculation process section 25 may find a vegetation index other than the normalized difference vegetation index NDVI (e.g., ratio vegetation index (RVI) or green NDVI (GNDVI)) by using a pixel value R representing a red component in the visible range, a pixel value G representing a green component in the visible range, a pixel value B representing a blue component in the visible range, and a pixel value IR representing a component in the near-infrared range.

<Processing for Finding Vegetation Index>

Fig. 16 is a flowchart describing an example of a process for the vegetation inspection system 11 to find a vegetation index.

In step S11, the weather information acquisition section 23 acquires weather information in the field 14 and supplies the information to the imaging control section 24.

In step S12, the imaging control section 24 decides whether or not it is time for the imaging apparatuses 12-1 and 12-2 to image the field 14. For example, when the weather is cloudy to such an extent that the field 14 remains unaffected by the angle L(θ,φ) of the light shining direction from the sun on the basis of weather information supplied from the weather information acquisition section 23 in step S11, the imaging control section 24 decides that it is time to perform imaging. Alternatively, the imaging control section 24 may decide whether or not it is time to perform imaging on the basis of time information as described above.

In a case where the imaging control section 24 decides in step S12 that it is not time for the imaging apparatuses 12-1 and 12-2 to take images of the field 14, the process returns to step S11 to repeat the same processes from here onward. On the other hand, in a case where the imaging control section 24 decides in step S12 that it is time for the imaging apparatuses 12-1 and 12-2 to take images of the field 14, the process proceeds to step S13.

In step S13, the imaging control section 24 sends, to the imaging apparatuses 12-1 and 12-2, an imaging command instructing to perform imaging via the communication section 21, and the imaging apparatuses 12-1 and 12-2 take images of the field 14 in accordance with the imaging command. Then, each of the imaging apparatuses 12-1 and 12-2 sends the taken image of the field 14, and the communication section 21 acquires the image data of these images and accumulates the data in the data server 22.

In step S14, the vegetation index calculation section 31 reads two images worth of image data to be processed from the data server 22 and computes the above Formula (1), finding the normalized difference vegetation index NDVI for each pixel making up each image and generating two NDVI images.

In step S15, the lens distortion correction section 32 performs a process of correcting lens distortion on each of the two NDVI images generated by the vegetation index calculation section 31 in step S14, generating two NDVI images with corrected lens distortion.

In step S16, the addition process section 33 performs a process of taking the arithmetic mean of the two NDVI images whose lens distortion has been corrected by the lens distortion correction section 32 in step S15, generating a single NDVI image with cancelled component dependent upon the imaging direction angle.

In step S17, the cancellation process section 34 adds the areas whose grain angles are opposite to each other in the NDVI image generated by the addition process section 33 in step S16, cancelling the component dependent upon the grain angle.

In step S18, the integration process section 35 divides the NDVI image whose component dependent upon the grain angle has been cancelled by the cancellation process section 34 in step S17 into grain areas and integrates the image.

Then, after the process in step S18, the process returns to step S11 to repeat the similar processes from here onward.

As described above, the vegetation inspection system 11 makes it possible to acquire an NDVI image made up of the normalized difference vegetation index NDVI of the entire field 14 by cancelling the component dependent upon the grain angle and cancelling the component dependent upon the imaging direction angle. Therefore, it is possible to inspect vegetation of the lawn raised in the field 14 by eliminating the effects dependent upon the grain angle, imaging direction angle, and shining direction angle using the vegetation inspection system 11.

It should be noted that, in order to cancel the effects dependent upon the grain angle, imaging direction angle, and shining direction angle in the present embodiment, an arithmetic mean process is performed by using images whose corresponding positive and negative components of the respective angles completely conflict with each other for ease of comprehension. For the imaging direction, for example, an arithmetic mean process is performed by using images from two directions at the imaging direction angles C(c,d) and C(-c,d). In contrast, actually, even if images with completely conflicting positive and negative components of the angles are not used, and, for example, as long as the components conflict with each other within a given reference value such as 5% or 10%, it is possible to reduce the angle-dependent effects by using such images. Further, the grain angle, imaging direction angle, and shining direction angle are merely examples of angles related to inspection of the field 14, and other angles may also be used.

It should be noted that although a configuration for taking two images with the two imaging apparatuses 12-1 and 12-2 has been described in the present embodiment, for example, the normalized difference vegetation index NDVI may be calculated from two images taken successively by moving the single imaging apparatus 12. For example, imaging may be performed at an arbitrary position by mounting the imaging apparatus 12 to an unmanned aerial vehicle (UAV). Further, two or more imaging apparatuses 12 may be used, and, for example, the normalized difference vegetation index NDVI may be calculated from four images taken from east-west and north-south directions using the four imaging apparatuses 12.

Further, it is not necessary to provide the lens distortion correction section 32 if images free from lens distortion can be taken. It should be noted that in addition to a configuration in which the imaging apparatuses 12 and the vegetation inspection apparatus 13 are connected via a communication network, images may be supplied via a recording medium, for example, with the imaging apparatuses 12 and the vegetation inspection apparatus 13 put offline. In this case, there is no need to provide the data server 22. Also, the imaging apparatuses 12 and the vegetation inspection apparatus 13 may be configured as an integral apparatus.

Further, for example, the imaging apparatuses 12 can find a vegetation index and send the index to the vegetation inspection apparatus 13, and the vegetation inspection apparatus 13 can perform a process of eliminating angle-dependent effects. Also, the process of eliminating angle-dependent effects, for example, is not limited to the vertical direction as illustrated by the grain angle P(0,0), the imaging direction angle C(0,0), and the shining direction angle L(0,0) in Fig. 3, and it is only necessary for these angles to be at fixed angles other than the vertical direction. That is, when vegetation is compared, it is only necessary to eliminate angle-dependent effects such that the same conditions are present at all times.

Further, the system in the present specification refers to the apparatus as a whole made up of a plurality of apparatuses.

It should be noted that the respective processes described with reference to the above flowchart need not necessarily be performed chronologically in accordance with the sequence described as a flowchart and include those that are performed in parallel or individually (e.g., parallel processes or object-based processes). Further, the program may be processed by a single central processing unit (CPU) or by a plurality of CPUs in a distributed manner.

Further, the above series of processes may be performed by hardware or software. In a case where the series of processes are performed by software, the program making up the software is installed from a program recording medium recording the program to a computer incorporated in dedicated hardware or, for example, to a general-purpose personal computer or other computer capable of performing various functions by installing various programs.

Fig. 17 is a block diagram illustrating a hardware configuration example of a computer for performing the above series of processes using a program.

In the computer, a CPU 101, a read only memory (ROM) 102, and a random access memory (RAM) 103 are connected to each other by a bus 104.

An input/output (I/O) interface 105 is further connected to the bus 104. An input section 106, an output section 107, a storage section 108, a communication section 109, and a drive 110 are connected to the I/O interface 105. The input section 106 includes a keyboard, a mouse, a microphone, and so on. The output section 107 includes a display, a speaker, and so on. The storage section 108 includes a hard disk, a non-volatile memory, and so on. The communication section 109 includes a network interface and so on. The drive 110 records information to or reads information from a removable recording medium 111 such as magnetic disk, optical disk, magneto-optical disk, or semiconductor memory.

In the computer configured as described above, the CPU 101 performs the above series of processes, for example, by loading the program stored in the storage section 108 into the RAM 103 via the I/O interface 105 and bus 104 for execution.

The program executed by the computer (CPU 101) is provided in a manner stored in, for example, a magnetic disk (including flexible disk), an optical disk (e.g., compact disk-read only memory (CD-ROM), digital versatile disk (DVD)), magneto-optical disk, or a removable medium 111 which is a packaged medium including semiconductor memory. Alternatively, the program is provided via a wired or wireless transmission medium such as local area network, the Internet, and digital satellite broadcasting.

Then, the program can be installed to the storage section 108 via the I/O interface 105 as the removable medium 111 is inserted into the drive 110. Alternatively, the program can be received by the communication section 109 via a wired or wireless transmission medium and installed to the storage section 108. In addition to the above, the program can be installed, in advance, to the ROM 102 or storage section 108.

It should be noted that the present embodiment is not limited to that described above and can be modified in various ways without departing from the gist of the present technology.

[Reference Signs List]

| 11 | Vegetation inspection system |
| 12-1, 12-2 | apparatuses |
| 13 | Vegetation inspection apparatus |
| 14 | Field |
| 21 | Communication section |
| 22 | Data server |
| 23 | Weather information acquisition section |

| 24 | Imaging control section |
| 25 | Calculation process section |
| 31 | Vegetation index calculation section |
| 32 | Lens distortion correction section |
| 33 | Addition process section |
| 34 | Cancellation process section |
| 35 | Integration process section |

**Claims**

1. A vegetation inspection apparatus (11) comprising:

   a calculation process section (25) configured to calculate an inspection value for inspecting plant vegetation by using imaging data having conflicting inspection-related-angle-dependent components such that the inspection-related-angle-dependent components included in imaging data acquired by imaging the plant vegetation are reduced; and
   an imaging control section (24) configured to control an imaging apparatus (12) for imaging the plant vegetation, wherein the imaging control section causes the imaging apparatus to image the plant vegetation at timings that are in accordance with imaging directions of imaging the plant vegetation and shining directions of light shone on the plant vegetation such that the inspection-related-angle-dependent components included in the acquired imaging data are reduced.

2. The vegetation inspection apparatus (11) of claim 1, wherein
   the calculation process section (25) reduces the angle-dependent components included in the imaging data in accordance with a growing direction (P) of the plant vegetation by performing a calculation process using images of two areas of a field (14) where azimuth angles (a, -a) of the growing direction (P) of the plant vegetation are opposite to each other.

3. The vegetation inspection apparatus (11) of claim 2, wherein
   the imaging data includes imaging values of respective wavelength ranges acquired on the basis of a sensor that has detection devices (12), arranged two-dimensionally, for detecting light in different wavelength ranges for the respective wavelength ranges.

4. The vegetation inspection apparatus (11) of claim 3, wherein
   the imaging data includes imaging values in respective red, green, blue, and near-infrared wavelength ranges.

5. The vegetation inspection apparatus (11) of claim 4, wherein
   the inspection value is a normalized difference vegetation index.

6. The vegetation inspection apparatus (11) of claim 1, wherein
   the calculation process section (25) reduces the angle-dependent component included in the imaging data in accordance with the imaging directions in which the plant vegetation is imaged by performing a calculation process using two pieces of the imaging data in which the azimuth angles of the imaging directions at the times of imaging the imaging data are in directions opposite to each other.

7. The vegetation inspection apparatus (11) of claim 1, wherein
   the calculation process section (25) reduces the angle-dependent component included in the imaging data in accordance with the shining directions of light shone on the plant vegetation by performing a calculation process using two pieces of the imaging data in which the azimuth angles of shining directions of light shone on the plant vegetation at the times of imaging the imaging data are in directions opposite to each other.

8. The vegetation inspection apparatus (11) of claim 1, further comprising:

   a weather information acquisition section (23) configured to acquire weather information indicating weather of a field where the plant vegetation is raised, wherein
   the imaging control section (24) causes the imaging apparatus (12) to image the plant when the weather is cloudy in the field.

9. The vegetation inspection apparatus (11) of claim 1, wherein
   the imaging data includes imaging values of respective wavelength ranges acquired on the basis of a sensor (12) that has detection devices, arranged two-dimensionally, for detecting light in different wavelength ranges for the respective wavelength ranges.

10. The vegetation inspection apparatus (11) of claim 9, wherein
    the imaging data includes imaging values in respective red, green, blue, and near-infrared wavelength ranges.

11. The vegetation inspection apparatus (11) of claim 10, wherein
    the inspection value is a normalized difference vegetation index.

**12.** A vegetation inspection method for calculating an inspection value for inspecting plant vegetation comprising:

controlling an imaging apparatus (12) for imaging the plant vegetation, wherein the imaging apparatus (12) images the plant vegetation at timings that are in accordance with imaging directions of imaging the plant vegetation and shining directions of light shone on the plant vegetation such that inspection-related-angle-dependent components included in the acquired imaging data are reduced; and
calculating an inspection value for inspecting plant vegetation by using the acquired imaging data.

**13.** A program causing a computer to function as:

a calculation process section (25) configured to calculate an inspection value for inspecting plant vegetation by using imaging data having conflicting inspection-related-angle-dependent components such that the inspection-related-angle-dependent components included in imaging data acquired by imaging the plant vegetation are reduced; and
an imaging control section (24) configured to control an imaging apparatus (12) for imaging the plant vegetation, wherein the imaging control section causes the imaging apparatus to image the plant vegetation at timings that are in accordance with imaging directions of imaging the plant vegetation and shining directions of light shone on the plant vegetation such that the inspection-related-angle-dependent components included in the acquired imaging data are reduced.

**Patentansprüche**

**1.** Vegetationsprüfvorrichtung (11), umfassend:

einen Berechnungsprozesssektor (25), der konfiguriert ist zum Berechnen eines Prüfwerts zum Prüfen einer Pflanzenvegetation, indem Bilderfassungsdaten verwendet werden, die prüfungsbezogene winkelabhängige Komponenten aufweisen, sodass die prüfungsbezogenen winkelabhängigen Komponenten verringert werden, die in einem Konflikt stehen und die in den Bilderfassungsdaten enthalten sind, die durch eine Bilderfassung der Pflanzenvegetation erlangt wurden; und
einen Bilderfassungssteuersektor (24), der konfiguriert ist zum Steuern einer Bilderfassungsvorrichtung (12) für eine Bilderfassung der Pflanzenvegetation, wobei der Bilderfassungssteuersektor die Bilderfassungsvorrichtung veranlasst, eine Bilderfassung der Pflanzenvegetation zu Zeitpunkten auszuführen, die mit den Bilderfassungsrichtungen der Bilderfassung der Pflanzenvegetation und mit Beleuchtungsrichtungen des Lichts übereinstimmen, das die Pflanzenvegetation beleuchtet, sodass die prüfungsbezogenen winkelabhängigen Komponenten verringert werden, die in den erlangten Bilderfassungsdaten enthalten sind.

**2.** Vegetationsprüfvorrichtung (11) nach Anspruch 1, wobei,
der Berechnungsprozesssektor (25) die winkelabhängigen Komponenten, die in den Bilderfassungsdaten enthalten sind, gemäß einer Wachstumsrichtung (P) der Pflanzenvegetation verringert, indem ein Berechnungsprozess ausgeführt wird, der Bilder von zwei Bereichen eines Feldes (14) verwendet, in denen die Azimutwinkel (a, -a) der Wachstumsrichtung (P) der Pflanzenvegetation zueinander entgegengesetzt sind.

**3.** Vegetationsprüfvorrichtung (11) nach Anspruch 2, wobei
die Bilderfassungsdaten Bilderfassungswerte von entsprechenden Wellenlängenbereichen enthalten, die auf der Grundlage eines Sensors erlangt werden, der verschiedene Erkennungseinheiten (12) aufweist, die zweidimensional angeordnet sind, um Licht in verschiedenen Wellenlängenbereichen für die entsprechenden Wellenlängenbereiche zu erkennen.

**4.** Vegetationsprüfvorrichtung (11) nach Anspruch 3, wobei
die Bilderfassungsdaten Bilderfassungswerte in entsprechenden Rot-, Grün-, Blau- und Nahinfrarot-Wellenlängenbereichen enthalten.

**5.** Vegetationsprüfvorrichtung (11) nach Anspruch 4, wobei
der Prüfwert ein normalisierter Differenzvegetationsindex ist.

**6.** Vegetationsprüfvorrichtung (11) nach Anspruch 1, wobei
der Berechnungsprozesssektor (25) die winkelabhängigen Komponenten, die in den Bilderfassungsdaten enthalten sind, gemäß der Bilderfassungsrichtungen verringert, in denen die Bilderfassung der Pflanzenvegetation ausgeführt wird, indem ein Berechnungsprozess ausgeführt wird, der zwei Teile der Bilderfassungsdaten verwendet, in denen sich die Azimutwinkel der Bilderfassungsrichtungen zu den Zeitpunkten der Bilderfassung der Bilderfassungsdaten in zueinander entgegengesetzten Richtungen befinden.

7. Vegetationsprüfvorrichtung (11) nach Anspruch 1, wobei der Berechnungsprozesssektor (25) die winkelabhängigen Komponenten, die in den Bilderfassungsdaten enthalten sind, gemäß der Beleuchtungsrichtungen des Lichts verringert, das die Pflanzenvegetation beleuchtet, indem ein Berechnungsprozess ausgeführt wird, der zwei Teile der Bilderfassungsdaten verwendet, in denen sich die Azimutwinkel der Beleuchtungsrichtungen des Lichts, das die Pflanzenvegetation beleuchtet, zu den Zeitpunkten der Bilderfassung der Bilderfassungsdaten in zueinander entgegengesetzten Richtungen befinden.

8. Vegetationsprüfvorrichtung (11) nach Anspruch 1, das außerdem umfasst:
einen Wetterinformationserlangungssektor (23), der konfiguriert ist zum Erlangen von Wetterinformationen, die das Wetter eines Feldes anzeigen, wo die Pflanzenvegetation aufgezogen wird, wobei der Bilderfassungssteuersektor (24) die Bilderfassungsvorrichtung (12) veranlasst, die Bilderfassung der Pflanze auszuführen, wenn das Wetter in dem Feld bewölkt ist.

9. Vegetationsprüfvorrichtung (11) nach Anspruch 1, wobei
die Bilderfassungsdaten Bilderfassungswerte von entsprechenden Wellenlängenbereichen enthalten, die auf der Grundlage eines Sensors (12) erlangt werden, der Erkennungseinheiten aufweist, die zweidimensional angeordnet sind, um Licht in verschiedenen Wellenlängenbereichen für die entsprechenden Wellenlängenbereiche zu erkennen.

10. Vegetationsprüfvorrichtung (11) nach Anspruch 9, wobei
die Bilderfassungsdaten Bilderfassungswerte in entsprechenden Rot-, Grün-, Blau- und Nahinfrarot-Wellenlängenbereichen enthalten.

11. Vegetationsprüfvorrichtung (11) nach Anspruch 10, wobei
der Prüfwert ein normalisierter Differenzvegetationsindex ist.

12. Vegetationsprüfverfahren zum Berechnen eines Prüfwerts für eine Prüfung einer Pflanzenvegetation, umfassend:

Steuern einer Bilderfassungsvorrichtung (12) für eine Bilderfassung der Pflanzenvegetation, wobei die Bilderfassungsvorrichtung (12) eine Bilderfassung der Pflanzenvegetation zu Zeitpunkten ausführt, die mit den Bilderfassungsrichtungen der Bilderfassung der Pflanzenvegetation und mit Beleuchtungsrichtungen des Lichts übereinstimmen, das die Pflanzenvegetation beleuchtet, sodass die prüfungsbezogenen winkelabhängigen Komponenten verringert werden, die in den erlangten Bilderfassungsdaten enthalten sind; und
Berechnen eines Prüfwerts für eine Prüfung einer Pflanzenvegetation, indem die erlangten Bilderfassungsdaten verwendet werden.

13. Programm, das einen Computer veranlasst, um zu funktionieren als:

ein Berechnungsprozesssektor (25), der konfiguriert ist zum Berechnen eines Prüfwerts zum Prüfen einer Pflanzenvegetation, indem Bilderfassungsdaten verwendet werden, die prüfungsbezogene winkelabhängige Komponenten aufweisen, die in einem Konflikt stehen, sodass die prüfungsbezogenen winkelabhängigen Komponenten verringert werden, die in den Bilderfassungsdaten enthalten sind, die durch eine Bilderfassung der Pflanzenvegetation erlangt wurden; und
einen Bilderfassungssteuersektor (24), der konfiguriert ist zum Steuern einer Bilderfassungsvorrichtung (12) für eine Bilderfassung der Pflanzenvegetation, wobei der Bilderfassungssteuersektor die Bilderfassungsvorrichtung veranlasst, eine Bilderfassung der Pflanzenvegetation zu Zeitpunkten auszuführen, die mit den Bilderfassungsrichtungen der Bilderfassung der Pflanzenvegetation und mit Beleuchtungsrichtungen des Lichts übereinstimmen, das die Pflanzenvegetation beleuchtet, sodass die prüfungsbezogenen winkelabhängigen Komponenten verringert werden, die in den erlangten Bilderfassungsdaten enthalten sind.

**Revendications**

1. Appareil d'inspection de végétation (11) comprenant :

une section de traitement de calcul (25) configurée pour calculer une valeur d'inspection en vue d'inspecter la végétation de plantes en utilisant des données d'imagerie ayant des composantes dépendantes de l'angle associé à l'inspection contradictoires de telle sorte que les composantes dépendantes de l'angle associé à l'inspection contenues dans des données d'imagerie acquises par imagerie de la végétation de plantes sont réduites ; et
une section de commande d'imagerie (24) configurée pour commander un appareil d'imagerie (12) en vue d'imager la végétation, la section de commande d'imagerie conduisant l'appareil d'imagerie à imager la végétation de plantes à des moments qui correspondent à des directions d'imagerie de la végétation de plantes et des directions d'éclairement par la lumière brillant sur la végétation de plantes de telle sorte que les composantes dépendantes de l'angle associé à l'inspection contenues dans les données d'imagerie acquises sont réduites.

2. Appareil d'inspection de végétation (11) de la revendication 1, dans lequel
la section de traitement de calcul (25) réduit les composantes dépendantes de l'angle contenues dans les données d'imagerie en fonction d'une direction de croissance (P) de la végétation de plantes en effectuant un traitement de calcul en utilisant des images de deux zones d'un champ (14) où des angles d'azimut (a, -a) de la direction de croissance (P) de la végétation de plantes sont opposés l'un à l'autre.

3. Appareil d'inspection de végétation (11) de la revendication 2, dans lequel
les données d'imagerie comportent des valeurs d'imagerie de gammes respectives de longueurs d'onde acquises sur la base d'un capteur qui a des dispositifs de détection (12), disposés bidimensionnellement, pour détecter la lumière dans différentes gammes de longueurs d'onde pour les gammes respectives de longueurs d'onde.

4. Appareil d'inspection de végétation (11) de la revendication 3, dans lequel
les données d'imagerie comportent des valeurs d'imagerie dans des gammes respectives de longueurs d'onde rouges, vertes, bleues et dans le proche infrarouge.

5. Appareil d'inspection de végétation (11) de la revendication 4, dans lequel
la valeur d'inspection est un indice différentiel normalisé de végétation.

6. Appareil d'inspection de végétation (11) de la revendication 1, dans lequel
la section de traitement de calcul (25) réduit la composante dépendante de l'angle contenue dans les données d'imagerie en fonction des directions d'imagerie dans lesquelles la végétation de plantes est imagée en effectuant un traitement de calcul en utilisant deux morceaux des données d'imagerie dans lesquels les angles d'azimut des directions d'imagerie aux moments de l'imagerie des données d'imagerie sont dans directions opposées l'une à l'autre.

7. Appareil d'inspection de végétation (11) de la revendication 1, dans lequel
la section de traitement de calcul (25) réduit la composante dépendante de l'angle contenue dans les données d'imagerie en fonction des directions d'éclairement par la lumière brillant sur la végétation de plantes en effectuant un traitement de calcul en utilisant deux morceaux des données d'image dans lesquels les angles d'azimut de directions d'éclairement par la lumière brillant sur la végétation de plantes aux moments de l'imagerie des données d'imagerie sont dans des directions opposées l'une à l'autre.

8. Appareil d'inspection de végétation (11) de la revendication 1, comprenant en outre :

une section d'acquisition d'informations météorologiques (23) configurée pour acquérir des informations météorologiques indiquant la météo d'un champ où la végétation de plantes est cultivée, dans lequel
la section de commande d'imagerie (24) conduit l'appareil d'imagerie (12) à imager la plante quand la météo est nuageuse dans le champ.

9. Appareil d'inspection de végétation (11) de la revendication 1, dans lequel
les données d'imagerie comportent des valeurs d'imagerie de gammes respectives de longueurs d'onde acquises sur la base d'un capteur (12) qui a des dispositifs de détection, disposés bidimensionnellement, pour détecter la lumière dans différentes gammes de longueurs d'onde pour les gammes respectives de longueurs d'onde.

10. Appareil d'inspection de végétation (11) de la revendication 9, dans lequel
les données d'imagerie comportent des valeurs d'imagerie dans des gammes respectives de longueurs d'onde rouges, vertes, bleues et dans le proche infrarouge.

11. Appareil d'inspection de végétation (11) de la revendication 10, dans lequel
la valeur d'inspection est un indice différentiel normalisé de végétation.

12. Procédé d'inspection de végétation destiné à calculer une valeur d'inspection en vue d'inspecter la végétation de plantes comprenant :

la commande d'un appareil d'imagerie (12) en vue d'imager la végétation de plantes, l'appareil d'imagerie (12) imageant la végétation de plantes à des moments qui correspondent à des directions d'imagerie de la végétation de plantes et des directions d'éclairement par la lumière brillant sur la végétation de plantes de telle sorte que des composantes dépendantes de l'angle associé à l'inspection contenues dans les données d'imagerie acquises sont réduites ; et

le calcul d'une valeur d'inspection en vue d'inspecter la végétation de plantes au moyen des données d'imagerie acquises.

13. Programme conduisant un ordinateur à fonctionner comme :

une section de traitement de calcul (25) configurée pour calculer une valeur d'inspection en vue d'inspecter la végétation de plantes en utilisant des données d'imagerie ayant des composantes dépendantes de l'angle associé à l'inspection contradictoires de telle sorte que les composantes dépendantes de l'angle associé à l'inspection contenues dans des données d'imagerie acquises par imagerie de la végétation de plantes sont réduites ; et
une section de commande d'imagerie (24) configurée pour commander un appareil d'imagerie (12) en vue d'imager la végétation, la section de commande d'imagerie conduisant l'appareil d'imagerie à imager la végétation de plantes à des moments qui correspondent à des directions d'imagerie de la végétation de plantes et des directions d'éclairement par la lumière brillant sur la végétation de plantes de telle sorte que les composantes dépendantes de l'angle associé à l'inspection contenues dans les données d'imagerie acquises sont réduites.

FIG.1

# ＦＩＧ．2

# F I G . 3

L (0, 0)

C (0, 0)

P (0, 0)

# F I G . 4

$$\begin{array}{|c|}\hline P\,(0,\,0)\\ C\,(0,\,0)\\ L\,(e,\,f)\\\hline\end{array} + \begin{array}{|c|}\hline P\,(0,\,0)\\ C\,(0,\,0)\\ L\,(-e,\,f)\\\hline\end{array} = \begin{array}{|c|}\hline P\,(0,\,0)\\ C\,(0,\,0)\\ L\,(0,\,0)\\\hline\end{array}$$

# F I G . 5

| P (0, 0) | | P (0, 0) | | P (0, 0) |
|---|---|---|---|---|
| C (c, d) | + | C (−c, d) | = | C (0, 0) |
| L (0, 0) | | L (0, 0) | | L (0, 0) |

# FIG.6

(−e, f)

(e, f)

(c, d)

(−c, d)

$$
\begin{array}{|c|}
\hline
\text{P} (0, 0) \\
\text{C} (c, d) \\
\text{L} (e, f) \\
\hline
\end{array}
+
\begin{array}{|c|}
\hline
\text{P} (0, 0) \\
\text{C} (−c, d) \\
\text{L} (−e, f) \\
\hline
\end{array}
=
\begin{array}{|c|}
\hline
\text{P} (0, 0) \\
\text{C} (0, 0) \\
\text{L} (0, 0) \\
\hline
\end{array}
$$

# FIG.7

(a, b)

```
P (a, b)
C (0, 0)
L (0, 0)
```

+

(−a, b)

```
P (−a, b)
C (0, 0)
L (0, 0)
```

=

```
P (0, 0)
C (0, 0)
L (0, 0)
```

# FIG.8

P (a, b)
C (c, d)
L (0, 0)

+

P (−a, b)
C (−c, d)
L (0, 0)

=

P (0, 0)
C (0, 0)
L (0, 0)

EP 3 287 003 B1

F I G . 9

FIG.10

P (0, 0)
C (0, 0)
L (0, 0)

# FIG.11

# FIG.12

(a, b)

```
P(a, b)
C(0, 0)
L(0, 0)
```

+

(−a, b)

```
P(−a, b)
C(0, 0)
L(0, 0)
```

=

```
P(0, 0)
C(0, 0)
L(0, 0)
```

EP 3 287 003 B1

# FIG.13

| P (a, b) |
| C (c, d) |
| L (0, 0) |

+

| P (−a, b) |
| C (−c, d) |
| L (0, 0) |

=

| P (0, 0) |
| C (0, 0) |
| L (0, 0) |

EP 3 287 003 B1

# FIG.14

EP 3 287 003 B1

F I G . 1 5

P1

P3

P(a, b)
C(c, d)
L(0, 0)

P(a, b)
C(0, 0)
L(0, 0)

(a, b)

(a3, b3)
(a1, b1)
(a2, b2)
(a4, b4)

P5

P(a, b)
C(−c, d)
L(0, 0)

P2

P4

P(0, 0)
C(0, 0)
L(0, 0)

P6

EP 3 287 003 B1

# F I G . 1 6

START

ACQUIRE WEATHER INFORMATION — S11

TIME TO PERFORM IMAGING? — S12

NO

YES

TAKE IMAGES OF FIELD — S13

CALCULATE VEGETATION INDEX — S14

CORRECT LENS DISTORTION — S15

PERFORM ADDITION PROCESS — S16

PERFORM CALCELATION PROCESS — S17

PERFORM INTEGRATION PROCESS — S18

# FIG.17

EP 3 287 003 B1

**EP 3 287 003 B1**

## REFERENCES CITED IN THE DESCRIPTION

### Patent documents cited in the description

- JP 2003339238 A
- JP 2003009664 A